# EUROPEAN PATENT APPLICATION

(11) **EP 4 410 987 A1**
(43) Date of publication of application: **07.08.2024**
(21) Application number: 23154266.3
(22) Date of filing: 31.01.2023
(51) Int. Cl.: C12N 15/864, A61K 48/00, C12N 15/10, C40B 40/00, C07K 14/015, C12N 15/35

(54) **AN AAV9 CAPSID VARIANT FOR TARGETED GENE TRANSFER**

(71) Applicant: Universitätsklinikum Hamburg-Eppendorf, 20246 Hamburg (DE)
(72) Inventor: HUBER, Tobias B., 20246 Hamburg (DE); LU, Shun, 20246 Hamburg (DE); KÖRBELIN, Jakob, 20246 Hamburg (DE); WU, Guochao, 20246 Hamburg (DE); LIU, Shuya, 20246 Hamburg (DE)
(74) Representative: Stüven, Ralf

(57) **Abstract**

The present invention relates to a recombinant vector for targeting to kidney cells comprising a targeting peptide. Also, a nucleic acid encoding a recombinant adeno associated virus (AAV) capsid and a recombinant adeno associated capsid protein comprising the targeting peptide are contemplated. The invention moreover refers to recombinant vectors for use as a medicament and for the use in the treatment of kidney diseases.

## Description

### FIELD OF THE INVENTION

The present invention relates to a recombinant vector for targeting to kidney cells comprising a targeting peptide, which comprises the sequence GGNYXX. Also, a nucleic acid encoding a recombinant adeno associated virus (AAV) capsid and a recombinant AAV capsid protein comprising the targeting peptide are contemplated. The invention moreover refers to recombinant vectors for use as a medicament and for the use in the treatment of kidney diseases.

### BACKGROUND OF THE INVENTION

Gene therapy is a promising treatment option that uses therapeutic genes to treat or prevent diseases. Several gene therapy approaches are currently tested, including replacement of mutated genes that cause diseases by healthy non-mutated copies of the respective gene (gene replacement), inactivation of mutated genes that function improperly (gene silencing), or correcting gene mutations in somatic cells (gene editing).

In the field of gene therapy, specificity of recombinant vectors used to deliver therapeutic genes plays an important role, as the recombinant vectors must be able to allow such differentiation of the target cells. Non-specific vectors with broad tropism are prone to induce a large number of different cell types, which on the one hand leads to a decreased efficiency of the therapy as only a small number of the actual target cells are reached, and on the other hand, the transduction of non-target cells can lead to undesirable side effects. Particularly, kidney-targeted gene therapy is difficult because the kidney has a complex anatomical structure composed of various cell types in different renal compartments.

AAVs as vectors are promising candidates for use in clinical practice because they are considered to be relatively safe. From the clinical perspective, AAV vectors have successfully been applied to gene therapy in tissue such as liver and central nervous system. However, only little progresses in the kidney have been made. So for example, previous studies reported that AAV serotypes were not able to target kidney cells for gene therapy due to insufficient cell-specificity and inefficient transduction (Rubin et al. (2020)).

Thus, there is a need to improve recombinant vectors for gene therapy, in particular to improve the cell specificity of recombinant vectors. Especially there is a need to improve the targeting of kidney cells.

### OBJECTIVES AND SUMMARY OF THE INVENTION

The present invention solves the above-mentioned problem by using a recombinant vector for the targeted gene transfer into kidney cells. In particular, the invention relates to a novel peptide, which specifically binds kidney cells.

Accordingly, a first aspect of the invention refers to a recombinant vector for targeting to kidney cells, comprising a targeting peptide comprising the sequence GGNYXX (SEQ ID NO: 1), wherein each X at position 5 to 6 is independently selected from any naturally occurring amino acid.

Such a recombinant vector allows the specific targeting of the kidney cells. In particular, the recombinant vector enables the targeting cells of a subset of the thick ascending limb of Henle`s loop (TAL), being located adjacent to the juxtaglomerular apparatus (JGA) in the kidney. More particularly, the recombinant vector as described herein enables targeted gene transfer of kindey cells, such as cells of a subset of the thick ascending limb of Henle`s loop (TAL), being located adjacent to the juxtaglomerular apparatus (JGA) in the kidney. Therefore, such a recombinant vector enables the gene therapeutic treatment of defined diseases or conditions of the kidney.

The recombinant vector enables targeting of SLC12A1 positive TAL cells which are CLDN16 positive but CLDN10 negative.

In particular the recombinant vector enables targeting of SLC12A1+/CLDN16+/CLDN10- cells.

Typically, the targeting peptide comprises at most 20 amino acids, preferably at most 10 amino acids, more preferably at most 9 amino acids and even more preferably 8 amino acids.

In a specific embodiment, the targeting peptide comprises at most 7 amino acids. Accordingly, in one embodiment the targeting peptide has a sequence as set out in SEQ ID NO: 26, i.e. GGNYXXX, wherein each X at position 5 to 7 is independently selected from any naturally occurring amino acid.

In some embodiments, X at position 5 of the targeting peptide is G. Accordingly, in one embodiment the targeting peptide has a sequence as set out in SEQ ID NO: 24, i.e. GGNYGXX, wherein each X at position 6 to 7 is independently selected from any naturally occurring amino acid.
In some embodiments, X at position 7 of the targeting peptide is G. Accordingly, in one embodiment the targeting peptide has a sequence as set out in in SEQ ID NO: 25, i.e. GGNYXXG, wherein each X at position 5 to 6 is independently selected from any naturally occurring amino acid.

The targeting peptides as disclosed herein convey a high transduction specificity and efficiency for targeting a recombinant vector to kidney tissue, in particular for targeting cells of a subset of the thick ascending limb of Henle`s loop (TAL), being located adjacent to the juxtaglomerular apparatus (JGA) in the kidney.

In one embodiment, the targeting peptide comprises the sequence GGNYGLG (SEQ NO.: 2).

Typically, the targeting peptide is exposed to the surface of the recombinant vector. The exposure of the targeting sequence on the surface of the vector increases the specificity of the vector for the targeted cells.

In one embodiment, the recombinant vector is an adeno associated virus (AAV) vector.

AAVs are promising candidates for use in clinical practice because they are considered as relatively safe. AAV vectors are capable of introducing a transgene into a cell or tissue where it is expressed stable, leading to a longlasting and efficient expression. At the same time, these vectors do not possess any known pathogenic mechanism (Tenenbaum et al. (2003)). Therefore, they are particularly suitable for treating human kidney diseases.

In a particular embodiment, the AAV is serotype AAV9.

Typically, the targeting peptide is incorporated into at least one capsid protein of the recombinant vector.

In some embodiments the targeting peptide is incorporated into each capsid protein of the recombinant vector.

Incorporation of the targeting peptide in the capsid protein conveys the specificity of the vector to the target cell. Therefore, an incorporation of the targeting peptide into each capsid protein of the recombinant vector is advantageous due to its increased specific binding for the targeted kidney cells.

In particular, the recombinant vector contains 60 copies of the targeting peptide.

In a specific embodiment the targeting peptide is introduced into the capsid protein after an amino acid residue corresponding to A589 of (virion protein 1) VP1 of AAV9.

Another embodiment refers to a recombinant vector as described herein, wherein the recombinant vector comprises a transgene.
Such a transgene, being packed in the capsid may be transferred by the recombinant vector to the targeted cell where transcription and/or translation of the transgene may occur. For example a mutated gene, leading to an altered function or malfunctioning of the targeted cell may be replaced by non-mutated copies of the mutated gene in the targeted cell (gene replacement), a gene in the targeted cell may be inactivated (gene silencing) or a gene in somatic cells may be corrected (gene editing). Therefore, a transgene being packed in the capsid can help to treat/cure diseases caused by gene mutations of kidney cells.

Accordingly, another aspect of the invention refers to the recombinant vector as described herein for use as a medicament.

Yet another aspect of the invention refers to the recombinant vector as described herein for use in the treatment of kidney diseases, preferably disorders related to juxtaglomerular apparatus, abnormal blood pressure and abnormal glomerular filtration rate. Such a recombinant vector has therapeutic potential for treatments for diseases such as hypertension and functional disorders related to the renal glomeruli.

One embodiment refers to the recombinant vector for the use as described above, wherein the cells of the distal tubular epithelium adjacent to the kidney juxtaglomerular apparatus are specifically transduced.

A further aspect of the invention refers to a recombinant AAV capsid protein comprising the targeting peptide as described herein.

Another aspect of the invention refers to a nucleic acid encoding the recombinant AAV capsid protein as described herein.

### FIGURE LEGENDS

**Figure 1****. Selection of AAV9 heptamer peptide library in vivo**
   Screening of AAV9 mutant (W503A) with peptide insertion site at amino acid position A589 (VP1 numbering) using an *in vivo* selection protocol (Korbelin et al. (2017)) being adapted for the selection in kidney.
**Figure 2****. Fluorescence microscopy of transduced murine kidney cells, expressing GFP**
   Fluorescence microscopy of murine kidney cells. AAV9-JGA loaded with cDNA of green fluorescent protein (GFP) intravenously administrated in mice to visualize transduced cells. In the kidney, GFP can only be detected in the subset of the TAL adjacent to JGA. Fluorescence microscopy shows that a subset of the TAL adjacent to the JGA is transduced in the kidney by AAV9-JGA.

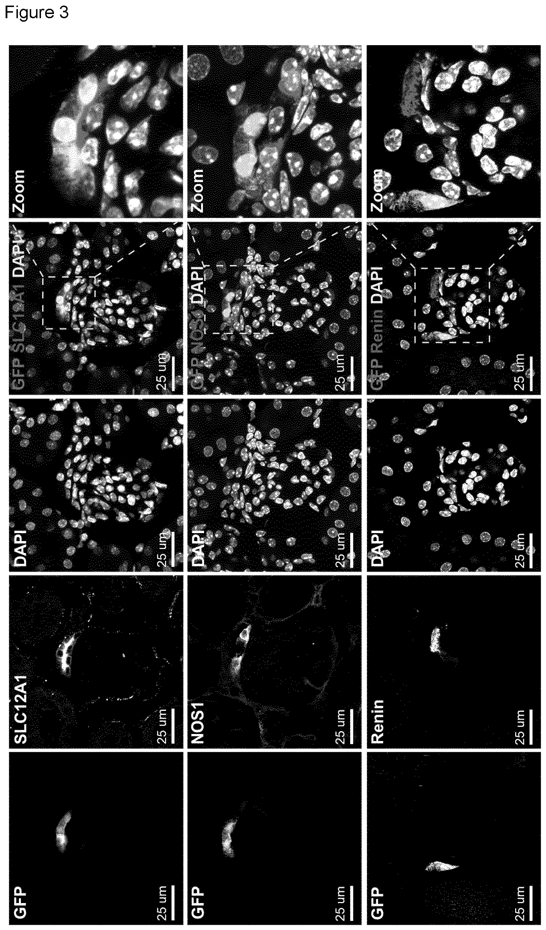

**Figure 3****. Fluorescence microscopy of AAV9-JGA transduced murine kidney cells, expressing GFP and stained with SLC12A1, NOS1 or Renin** Fluorescence microscopy of murine kidney cells transduced with AAV9-JGA. In the murine kidney, GFP was co-stained with SLC12A1, a marker for TAL. GFP-positive cells are located in JGA, close to the MD marked by NOS1 and the GC is marked by Renin. Nuclei are counterstained with DAPI.

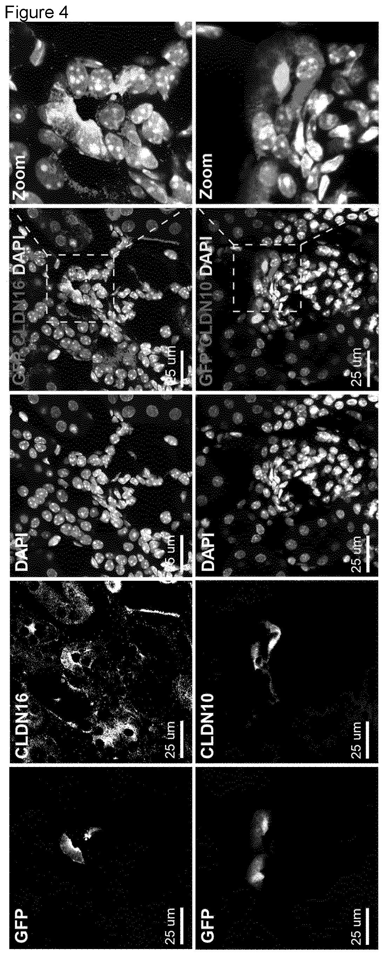

**Figure 4****. Fluorescence microscopy of AAV9-JGA transduced murine kidney cells, expressing GFP and stained with CLDN16 or CLDN10**
   Fluorescence microscopy of murine kidney cells transduced with AAV9-JGA. In the murine kidney, GFP was co-stained with CLDN16, a marker for a subset of TAL. GFP was not co-located with CLDN10, a marker for another subset of TAL. Nuclei are counterstained with DAPI.

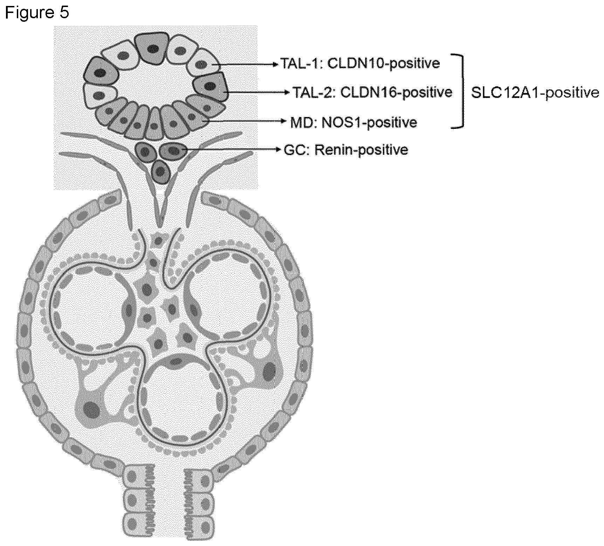

**Figure 5****. Structure and different cell types in the juxtaglomerular apparatus**
   Structure and different cell types in the juxtaglomerular apparatus (JGA) and the glomerulus. TAL: thick ascending limb of Henle's loop, MD: Macula densa, GC: Granular cells.

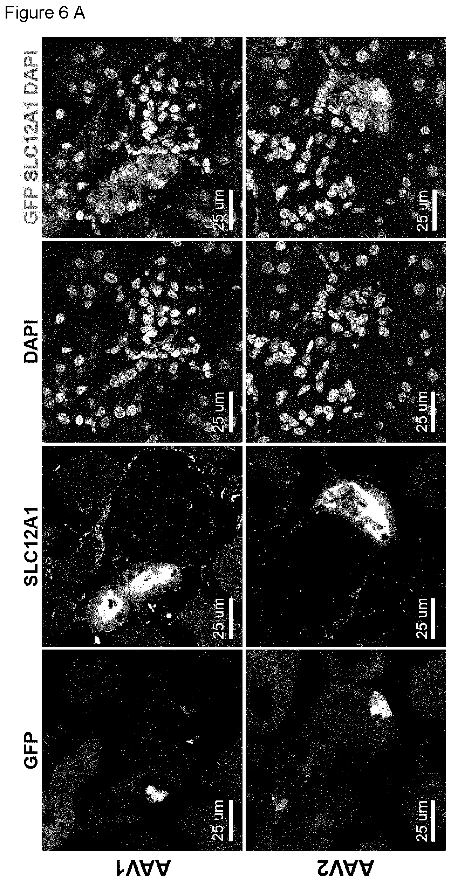

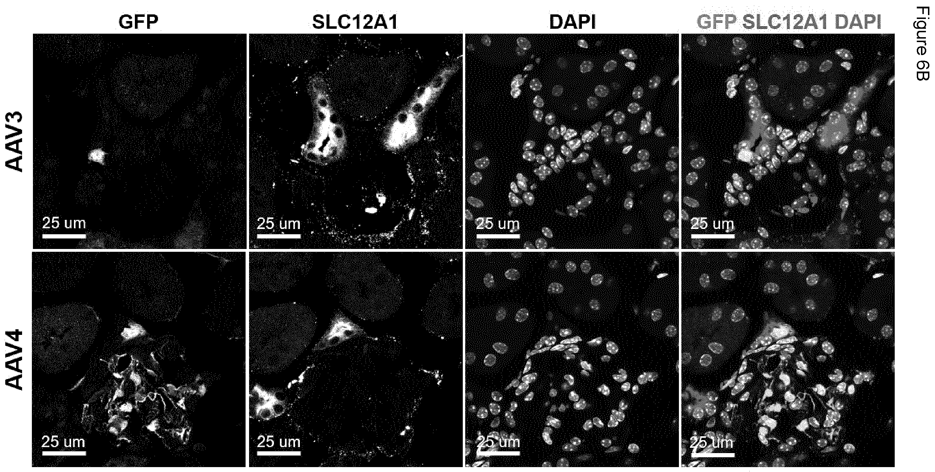

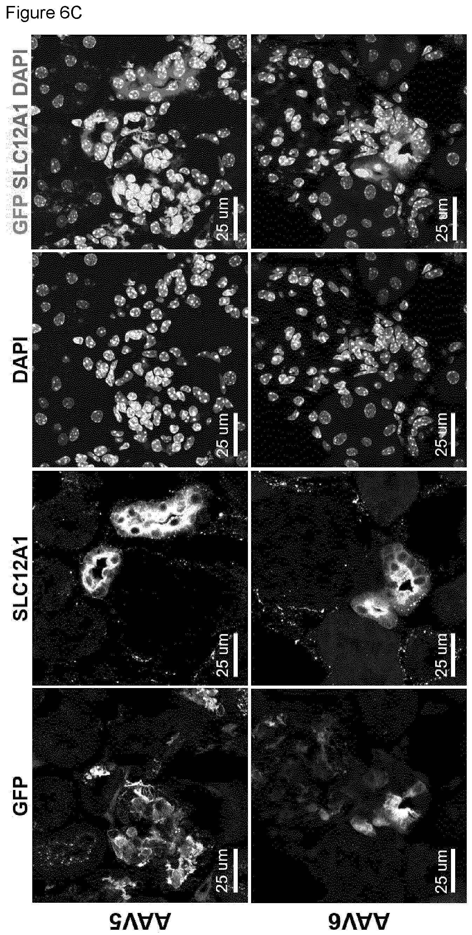

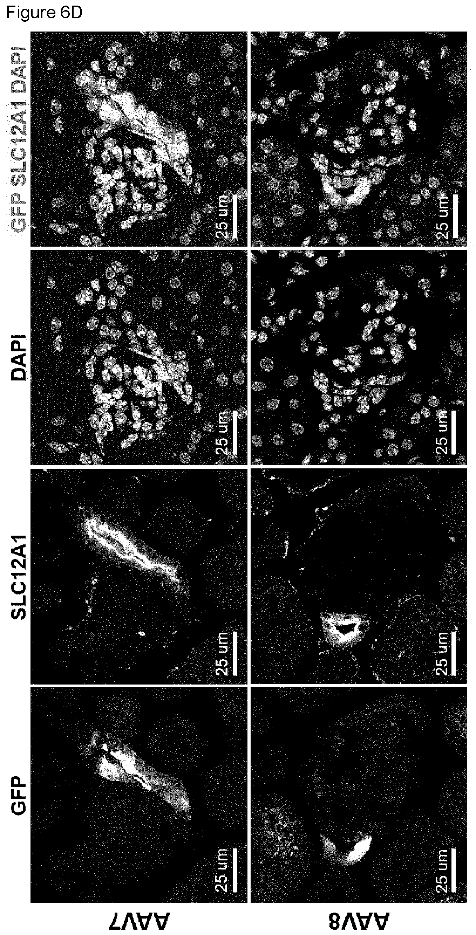

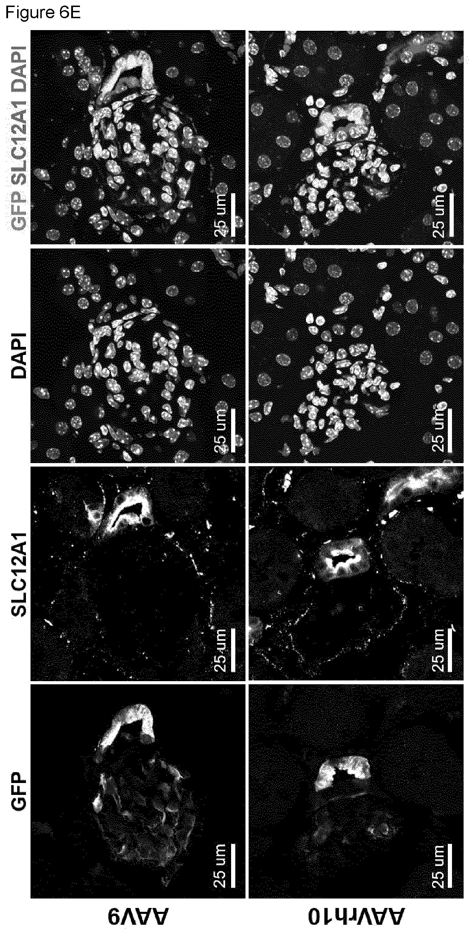

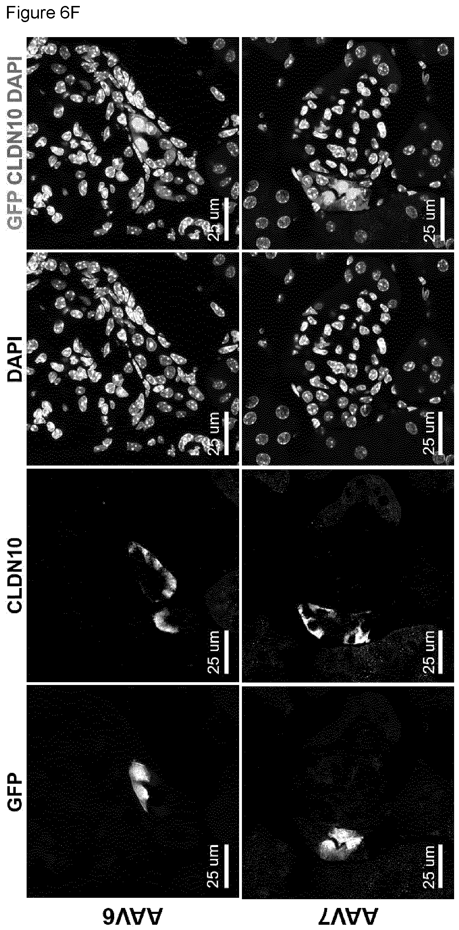

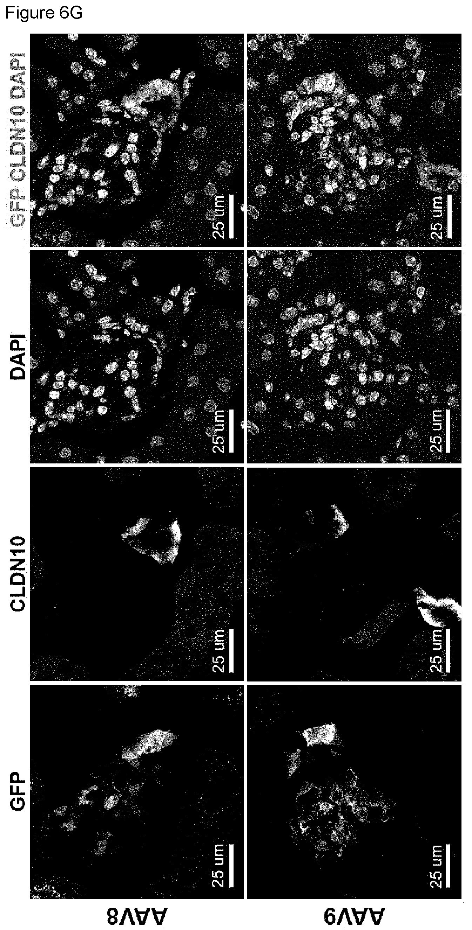

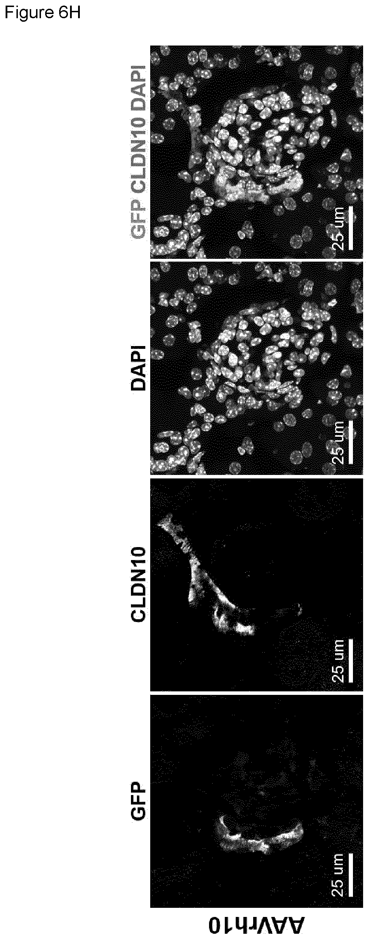

**Figure 6****. Fluorescence microscopy of various AAV serotypes transduced murine kidney cells, expressing GFP and stained with SLC12A1 or CLDN10**
   Fluorescence microscopy of murine kidney cells transduced with AAV1, AAV2, AAV3, AAV4, AAV5, AAV6, AAV7, AAV8, AAV9 or AAVrh10. The AAV loaded with GFP cDNA was intravenously injected in mice. In the murine kidney, GFP was co-stained with SLC12A1 (A-E), GFP was not co-located with CLDN10 (F-H).

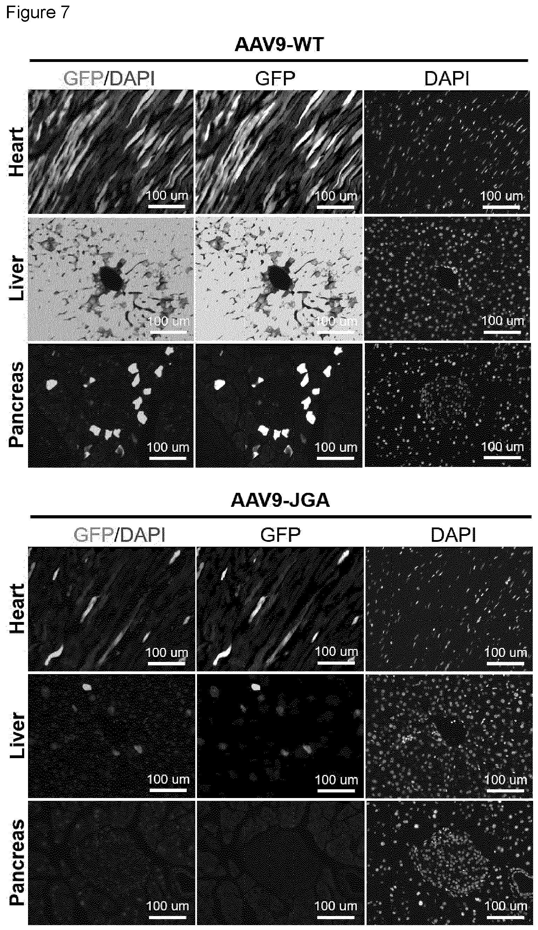

**Figure 7****. Fluorescence microscopy of transduced murine heart, liver and pancreas cells**
   Fluorescence microscopy of murine heart cells, liver cells and pancreas cells transduced with AAV9-JGA. GFP delivered by AAV9-JGA exhibits no obvious signal in the heart, liver, or pancreas, which are regarded as off-targets (left hand side), but GFP delivered by AAV9-WT shows strong signals in these organs (right hand side). Nuclei are counterstained with DAPI.

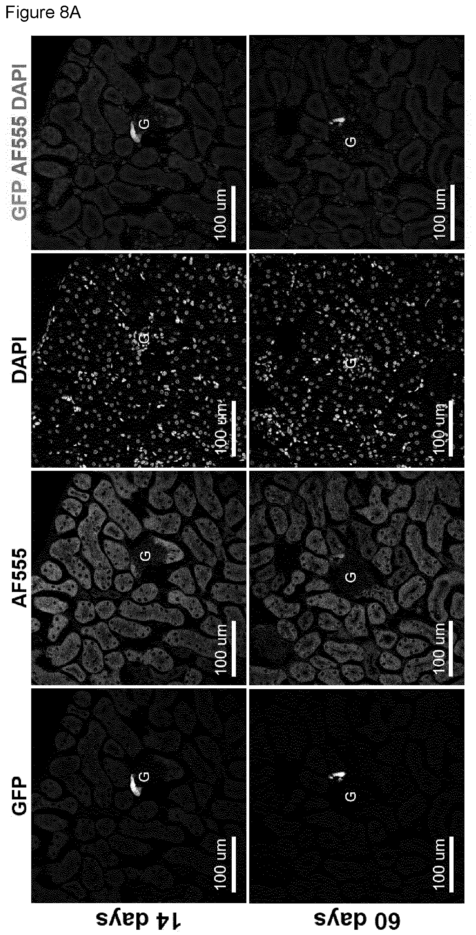

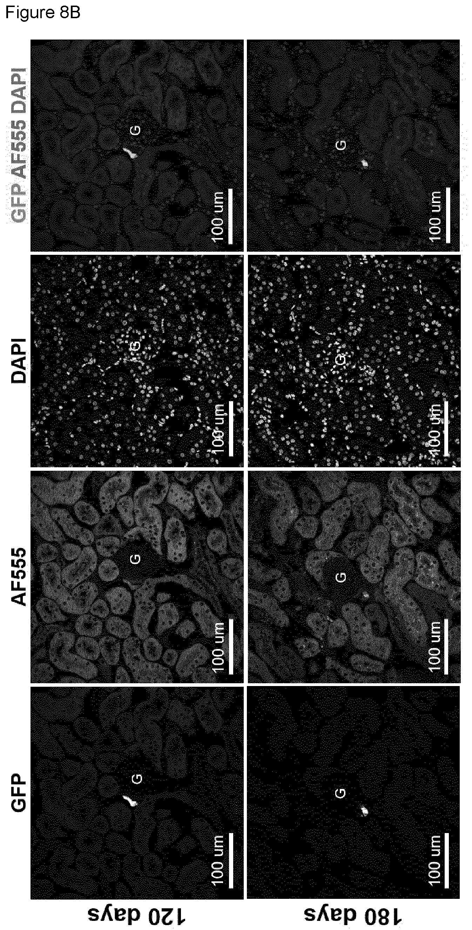

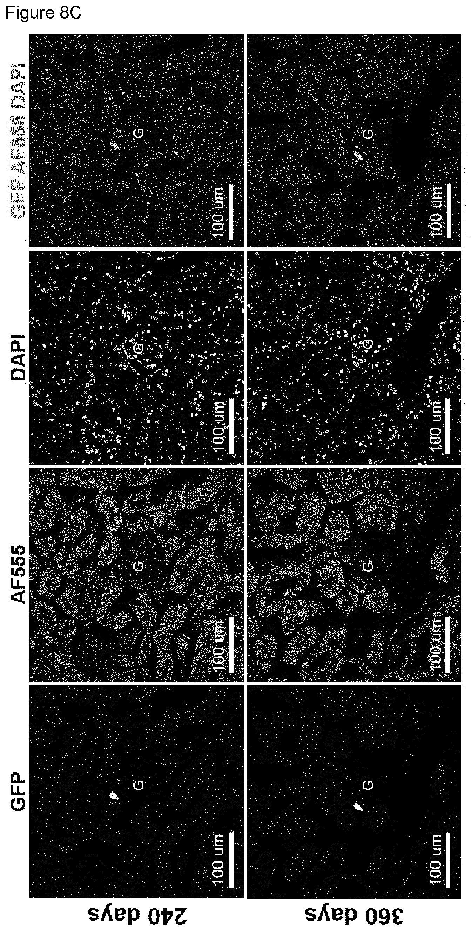

**Figure 8****. Fluorescence microscopy of AAV9-JGA transduced murine kidney cells, expressing GFP in long-term.**
   Fluorescence microscopy of murine kidney cells transduced with AAV9-JGA. In the murine kidney, GFP was detected at 14, 60, 120, 180, 240 and 360 days (A-C). Kidney structure is shown by its autofluorescence in AF555 channel and nuclei are counterstained with DAPI. AF555: Alex Flour 555, G: kidney glomerulus.

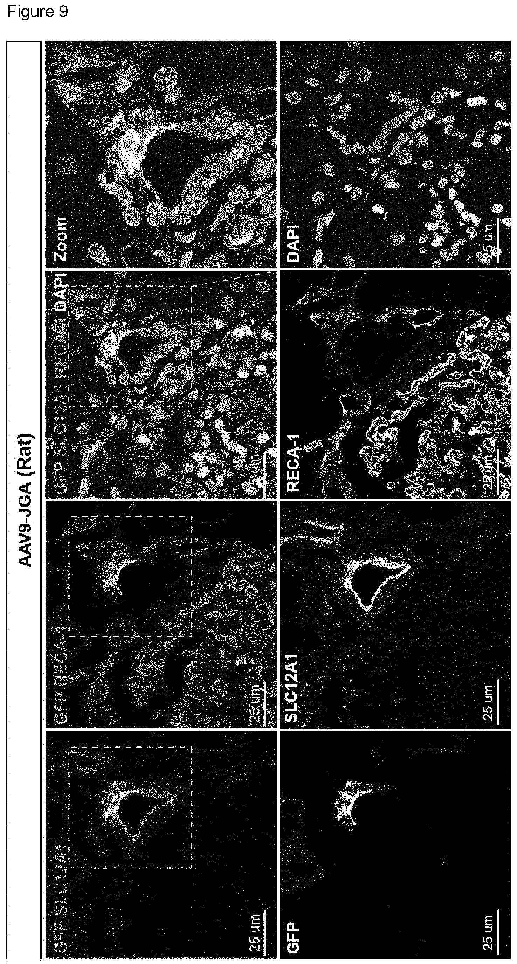

**Figure 9****. Fluorescence microscopy of transduced rat kidney cells.**
   Fluorescence microscopy of rat kidney cells transduced with AAV9-JGA. Staining with GFP/SLC12A1/RECA-1/DAPI. In the rats JGA, GFP was found in the subset of the TAL marked by SLC12A1, which is adjacent to the glomerulus visualized by the structure of glomerular endothelium marked by RECA-1. Cell nuclei were counterstained with DAPI.

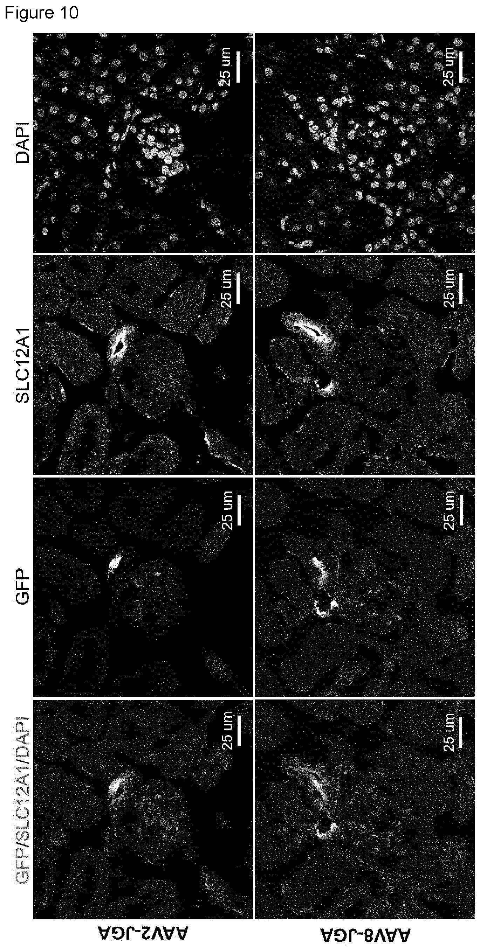

**Figure 10****. Fluorescence microscopy of AAV2-JGA and AAV8-JGA transduced murine kidney cells, expressing GFP and co-stained with SLC12A1**
   Fluorescence microscopy of murine kidney cells transduced with AAV2-JGA or AAV8-JGA. AAV2-JGA or AAV8-JGA loaded with GFP cDNA was intravenously injected in mice. GFP is co-stained with TAL marker SLC12A1, indicating that JGA peptide motif (GGNYGLG) in other AAV serotypes also specifically targets a subset of TAL cells in the kidney.

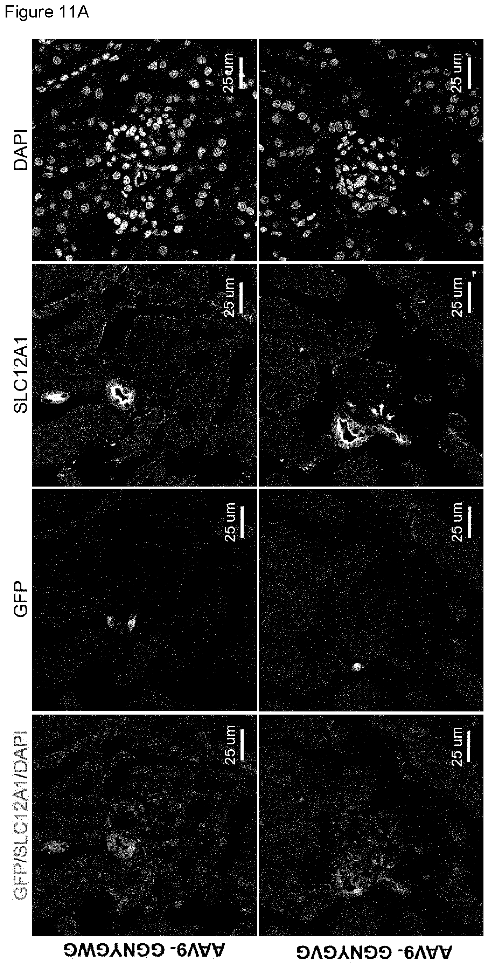

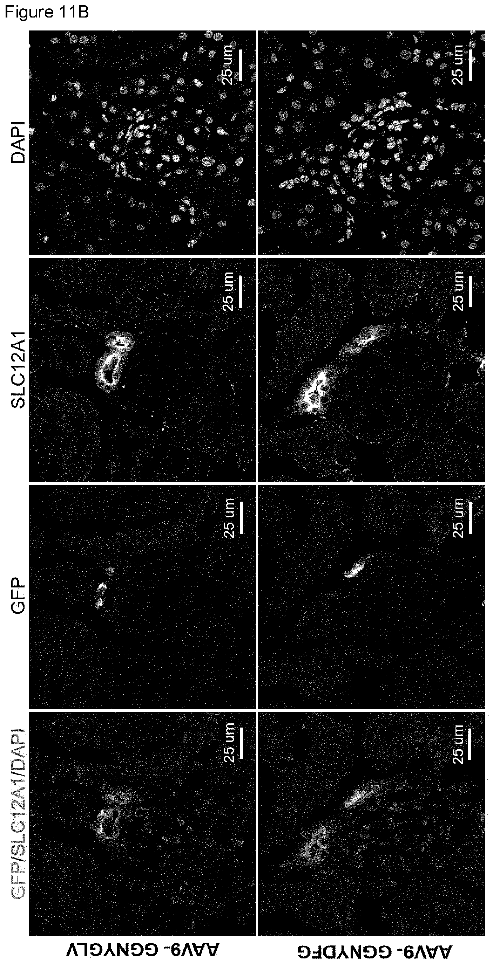

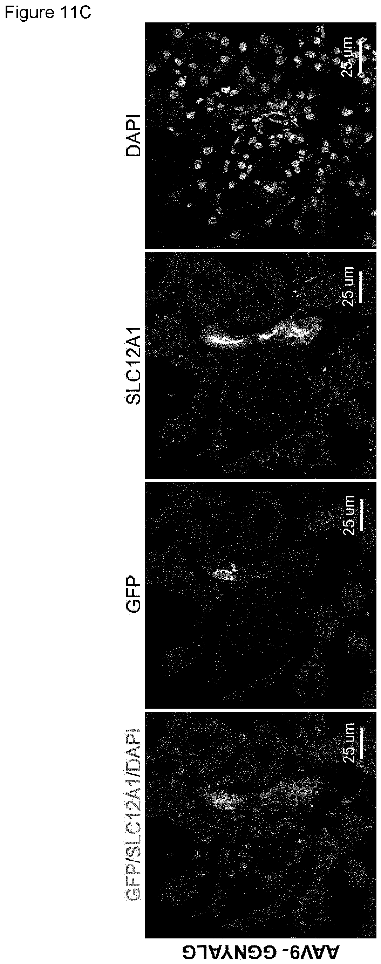

**Figure 11****. Fluorescence microscopy of AAV9-GGNYGXX or AAV9-GGNYXXG transduced murine kidney cells, expressing GFP and co-stained with SLC12A1**
   Fluorescence microscopy of murine kidney cells transduced with AAV9-GGNYGXX or AAV9-GGNYXXG. AAV9-GGNYGWG, AAV9-GGNYGVG, AAV9-GGNYGLV, AAV9-GGNYDFG, or AAV9-GGNYALG loaded with GFP cDNA was intravenously injected in mice (A-C). GFP is co-stained with TAL marker SLC12A1, indicating that peptide motif (GGNYGXX or GGNYXXG) specifically targets a subset of TAL cells in the kidney.

### DETAILED DESCRIPTION OF THE INVENTION

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±10%, and preferably of ±5%.

Technical terms are used by their common sense. If a specific meaning is conveyed to certain terms, definitions of terms will be given in the following in the context of which the terms are used.

The invention relates in a first aspect to a recombinant vector for cell specific targeting, in particular targeting to kidney cells, comprising a targeting peptide comprising the sequence GGNYXX, wherein each X at position 5 to 6 is independently selected from any naturally occurring amino acid.

A "vector" is any molecule or moiety suitable to be used in gene therapy that is capable to transfer polynucleotides to a targeted cell.

A vector according to the invention comprises a targeting peptide, which mediates the cell specificity of the vector for a targeted cell. Exemplary, a vector according to the invention may be a lipid nanoparticle, a virus, in preferably a retrovirus, an adenovirus or adeno-associated virus (AAV), more preferably an AAV.

Where the term "recombinant vector" is used, it indicates that the vector has been modified by the introduction of a heterologous nucleic acid or protein or by an alteration of a native nucleic acid or protein. Thus, a "recombinant vector" according to the invention is any vector as described above which is derived from the expression of genetically modified polynucleotides and/or which comprises genetically modified nucleotides or heterologous or modified proteins.

The term "transduction" as used herein refers to the process of introducing nucleic acids into mammalian cells by viral methods.

The term "targeting" refers to the specific direction of the recombinant vector as disclosed herein to a specific type of cell.

The delivery of genetic material to a target cell or tissue by means of recombinant vectors is known by the person skilled in the art and may be performed in a living organism (*in vivo*) or in cell culture (*in vitro*)*.*

The term "tissue" as used herein refers to an ensemble of similar cells and their extracellular matrix from the same origin that together carry out a specific function of an organ. Therefore, the present invention also relates to a recombinant vector for targeting to kidney tissue.

The term "targeting peptide" relates to a peptide being displayed on the recombinant vector, which mediates the cell specificity of the recombinant vector for a targeted cell by binding to the respective targeted cell. In particular, the targeting peptide according to the invention specifically binds to kidney cells.

A further embodiment refers to a recombinant vector for targeting to kidney cells, comprising a targeting peptide comprising the sequence GGNYXX, wherein each X at position 5 to 6 is independently selected from any naturally occurring amino acid and wherein the recombinant vector binds specifically to the kidney cells.

A further embodiment refers to a recombinant vector for targeting to kidney cells, comprising a targeting peptide comprising the sequence GGNYXX, wherein each X at position 5 to 6 is independently selected from any naturally occurring amino acid and wherein the recombinant vector binds specifically to a subset of the TAL which is adjacent to the JGA and locates at the opposite side of MD in the kidney.

The recombinant vector enables targeting of SLC12A1 positive TAL cells which are CLDN16 positive but CLDN10 negative. In particular the recombinant vector enables targeting of SLC12A1+/CLDN16+/CLDN10-cells.

The JGA is the most important functional structure in the kidney. It consists of the macula densa (MD) cells, the renin-secreting granular cells and extraglomerular mesangial cells (MC). The MD is an area of closely packed specialized cells lining the wall of the thick ascending limb of Henle's loop (TAL) next to the glomerulus. It functions as renal sensor elements that detect changes in distal tubular fluid composition. It regulates granular cells to release renin, an essential component of the renin-angiotensin-aldosterone system (RAAS), which regulates blood pressure and volume. Furthermore, it also known to transmit signals to the glomerular vascular elements to regulate the glomerular filtration rate (GFR) (Bell et al. (2003)).

Furthermore, in one embodiment the targeting peptide comprises at most 20 amino acids, preferably at most 10 amino acids, more preferably at most 9 amino acids and even more preferably 8 amino acids.

In some embodiments, the targeting peptide comprises at most 7 amino acids. Thus, in a specific embodiment the targeting peptide comprises the sequence as set out in SEQ ID NO: 26 (GGNYXXX). Exemplary targeting peptides are set out in SEQ ID NO: 2 to 23.

A further embodiment refers to the recombinant vector as described herein, where X at position 5 of the sequence of the targeting peptide is G. Thus, in a specific embodiment the targeting peptide comprises the sequence as set out in SEQ ID NO: 24 (GGNYGXX). Exemplary targeting peptides are set out in sequences SEQ ID NOs.: 2 to 19.

Another embodiment refers to the recombinant vector as described herein, where X at position 7 of the sequence of the targeting peptide is G. Thus, in a specific embodiment the targeting peptide comprises the sequence as set out in SEQ ID NO: 25 (GGNYXXG). Exemplary targeting peptides are set out in sequences SEQ ID NOs.: 2 to 10 and 20 to 22.

In some embodiments the X at the position 5 of the targeting peptide and the X at the position 7 of the targeting peptide is G. Thus, in a specific embodiment the targeting peptide comprises the sequence as set out in SEQ ID NO: 27 (GGNYGXG). Exemplary targeting peptides are set out in sequences SEQ ID NOs.: 2 to 10.

Another embodiment refers to the recombinant vector as described herein, where the targeting peptide comprises the sequence GGNYGLG (SEQ ID NO: 2).

In order to evaluate the cell specify a the targeting peptide for the targeted kidney cells a method as follows is presented, comprising the following steps:
a) providing a recombinant vector for targeting to kidney cells, comprising a targeting peptide comprising the sequence GGNYXX, wherein each X at position 5 to 6 is independently selected from any naturally occurring amino acid;
b) loading the recombinant vector of step a) with a gene encoding for a reporter protein;
c) contacting the recombinant vector b) with kidney cells (target cells) and cells of at least one cell type being different from the kidney cells (control cells);
d) analyzing the cells of step c) for a signal derived from the expression of the reporter gene, and
e) comparing a signal intensity of the signal obtained in step d) derived from the target cells with a signal intensity of control cells, wherein an increase of the signal in the target cells versus the control cells indicates that the targeting peptide is cell specific for kidney cells.

The reporter gene may for example be a gene encoding GFP or DsRed or luciferase. The analysis of the cells for the respective signal of the expressed reporter gene may be accomplished by fluorescence microscopy (Fig.5).

Yet another embodiment refers to the recombinant vector as described herein, where, the targeting peptide is exposed to the surface of the recombinant vector.

Yet another embodiment refers to a recombinant vector, wherein the vector is a viral vector.

Viral vectors are tools commonly used to deliver genetic material into targeted cells. Exemplary, but not limiting a viral vector may be a retrovirus, a lentivirus, an adenovirus or an adeno-associated virus.

In another specific embodiment, the vector is an adeno-associated virus (AAV) vector.

AAV are small non-enveloped viruses, which belong to the family of Parvoviridae and which comprise of a capsid with 60 proteins being arranged in a T = 1 icosahedral symmetry. The AAV genome consists of single stranded DNA of about 4.7 kilo bases. It comprises T-shaped inverted terminal repeats (ITRs) of 145 bases on each end and carries two open reading frames (ORF): rep and cap, which encode for functional and capsid proteins. The ORF rep encodes for four Rep proteins (Rep78, Rep68, Rep52 and Rep40). Rep 78 and Rep 68 have site-specific, single-strand endonuclease, DNA helicase, and ATP activities, respectively, which are responsible for DNA replication. Rep52 and Rep40 are responsible for packaging of DNA flanked by ITRs into capsids. The ORF cap encodes for three capsid proteins: VP1, VP2 and VP3. Additionally, the VP1 mRNA encodes for the assembly-activating protein (AAP) in a different reading frame. Based in the differences in the amino acid sequences of the capsid proteins, different AAV serotypes are distinguished, i.e. AAV1, AAV2, AAV3, AAV4, AAV5, AAV6, AAV7, AAV8, AAV9, AAV10, AAV11, AAV12, AAVrh10.

Thus, in a specific embodiment the AAV is selected from the group consisting of AAV1, AAV2, AAV3, AAV4, AAV5, AAV6, AAV7, AAV8, AAV9, AAV10, AAV11, AAV12, AAVrh10 or variants thereof or a combination thereof.

Typically, the serotype of the AAV is AAV9. AAV vectors of serotype 9 (AAV9) are of particular importance for clinical use. Systemically delivery of AAV9-based gene replacement therapy (onasemnogene abeparvovec) has been approved by the US Food and Drug Administration (FDA) for the treatment of spinal muscular atrophy.

A serotype corresponds to a variant subspecies of AAV which owing to its profile of expression of capsid surface antigens has a distinctive reactivity which can be used to distinguish it from other variant subspecies. Typically, a virus having a particular AAV serotype does not efficiently cross-react with neutralizing antibodies specific for any other AAV serotype.

Specific embodiments refer to an AAV comprising a targeting peptide having any one of the sequences 1 to 27. One embodiment refers to an AAV comprising a targeting peptide having the sequence set out in SEQ ID NO: 1. A more specific embodiment refers to an AAV comprising a targeting peptide having the sequence set out in SEQ ID NO: 2. One embodiment refers to an AAV comprising a targeting peptide having the sequence set out in SEQ ID NO: 28.

One embodiment refers to an AAV comprising a targeting peptide having the sequence set out in SEQ ID NO: 26. Specific embodiments refer to an AAV comprising a targeting peptide having the sequence selected form the group consisting of SEQ ID NO: 24, SEQ ID NO: 25, SEQ ID NO: 27.

Specific embodiments refer to an AAV9 comprising a targeting peptide having any one of the sequences 1 to 27. One embodiment refers to an AAV9 comprising a targeting peptide having the sequence set out in SEQ ID NO: 1. A more specific embodiment refers to an AAV9 comprising a targeting peptide having the sequence set out in SEQ ID NO: 2. One embodiment refers to an AAV9 comprising a targeting peptide having the sequence set out in SEQ ID NO: 28.

One embodiment refers to an AAV9 comprising a targeting peptide having the sequence set out in SEQ ID NO: 26. Specific embodiments refer to an AAV9 comprising a targeting peptide having the sequence selected form the group consisting of SEQ ID NO: 24, SEQ ID NO: 25, SEQ ID NO: 27.

In another embodiment of the recombinant vector, the targeting peptide is incorporated into at least one capsid protein of the recombinant vector.

For example, an AAV capsid is composed of three viral proteins (VPs): VP1, VP2, and VP3. The VP composition of the AAV capsid is estimated to be a molar ratio of 1:1:10, indicating that one AAV capsid typically has 5 VP1, 5 VP2, and 50 VP3 proteins. Therefore, in one embodiment the targeting peptide may be incorporated in the capsid proteins VP1 or VP2 or VP3 of an AAV vector.

In another embodiment, the targeting peptide is incorporated in each of the capsid proteins VP1 and VP2 and VP3.

Thus, in a particular embodiment, the targeting peptide of the recombinant vector is incorporated into each capsid protein of the recombinant vector.

In another embodiment, the recombinant vector contains 60 copies of the targeting peptide. As an AAV contains 60 capsid proteins this would mean, that each of the capsid proteins comprises at least one targeting peptide.

In some embodiments the targeting peptide is introduced into the capsid protein after an amino acid residue corresponding to A589 of (virion protein 1) VP1 of AAV9.

Some embodiments refer to chimeric AAVs, in which the capsid proteins stem from different AAV serotypes (e.g. VP1 stemming from AAV and VP2 und VP3 stemming from AAV9), or AAVs in which all capsid proteins contain sequence sections stemming from different AAV serotypes.

The recombinant vector of the invention may be used for cell specific delivering of at least one transgene. Accordingly, on embodiment refers to a recombinant vector comprising a transgene. Thus, some embodiments refer to an AAV comprising a transgene. In other words, the transgene is packaged within the capsid.

Typically, the transgene useful in the present invention will be a nucleotide sequence encoding a polypeptide of interest. The polypeptide may be an enzyme, ion channel, receptor (e.g., a GPCR), hormone, cytokine, chemokine, or an antibody or antibody fragment. Included within the scope of this invention is the insertion of one, two, or more transgenes.

In a specific embodiment, the transgene is a therapeutically active prophylactic active transgene.

As used herein, the term "therapeutically active" refers to any agent which can be used in treating, managing, or ameliorating symptoms associated with a disease or disorder, where the disease or disorder is associated with a function to be provided by a transgene. As used herein, the term "prophylactically active" refers to any agent which can be used in the prevention, delay, or slowing down of the progression of a disease or disorder, where the disease or disorder is associated with a function to be provided by a transgene.

In a specific embodiment the transgene comprises a reporter gene such as the cDNA of green fluorescent protein (GFP). After transduction of the reporter gene by the virus to the target cell, the reporter gene may be expressed in the target cell, enabling an identification of the successfully transduced cell.

Accordingly, a further aspect of the invention refers to a method of delivering a transgene to a cell, said method comprising contacting said cell with the recombinant vector, e.g. an AAV, as described herein.

Another aspect refers to a pharmaceutical composition for use in delivering a transgene to a target tissue of a subject in need thereof, said pharmaceutical composition comprising the recombinant vector, e.g. an AAV, as described herein.

A further aspect of the invention refers to a recombinant vector as described herein, for use as a medicament.

Another aspect of the invention refers to the recombinant vector as described herein for use in the treatment of kidney diseases, preferably disorders related to juxtaglomerular apparatus, abnormal blood pressure and abnormal glomerular filtration rate.

Thus, one embodiment refers to the use of the recombinant vector as described herein, wherein the cells of the distal tubular epithelium adjacent to the kidney juxtaglomerular apparatus are specifically transduced.

The glomerular filtration rate (GFR) is a blood test that determines the performance of a kidney. Within the kidney glomeruli act as filters for the blood, which help to remove waste and excess fluid from the blood. A GFR test determines how much blood passes through these glomeruli within a specified period of time.

Another aspect of the invention refers to a recombinant AAV capsid protein comprising the targeting peptide as described herein.

Yet another aspect of the invention refers to a nucleic acid encoding the recombinant AAV capsid protein described herein.

### Experiments

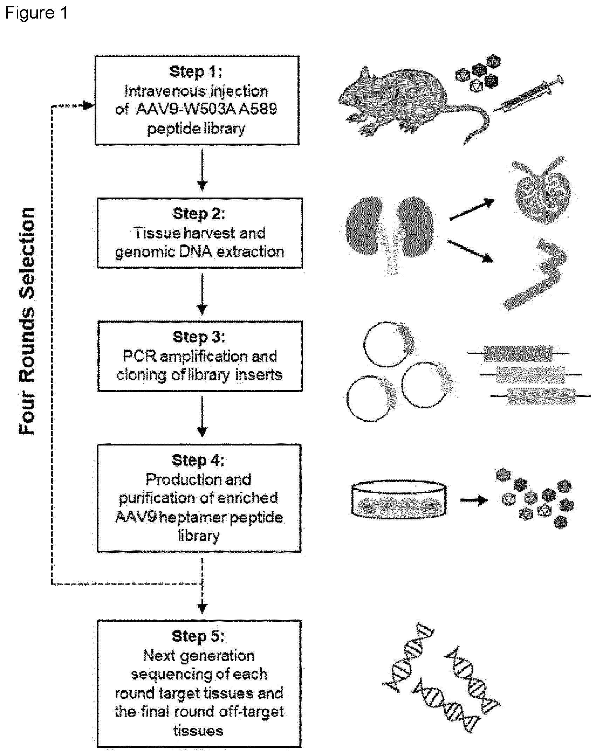

In the present invention, an AAV9 mutant (W503A) heptamer peptide library with peptide insertion site at amino acid position A589 (VP1 numbering) was screened using an in vivo selection protocol (4), which was adapted for the selection in the kidney **(****Figure 1****).**

A heptamer having particular specificity for the subset of the TAL adjacent to the JGA was peptide GGNYGLG. Based on this sequence, the recombinant viral vector AAV9-JGA was produced which expresses the peptide sequence GGNYGLG as a partial sequence of each virion protein (VP), resulting in a total of 60 peptide copies in the fully assembled viral capsids. Subsequently, vector AAV9-JGA was administered intravenously to mice and a clear specificity of the vector for the subset of the TAL adjacent to the JGA in the kidney in vivo.

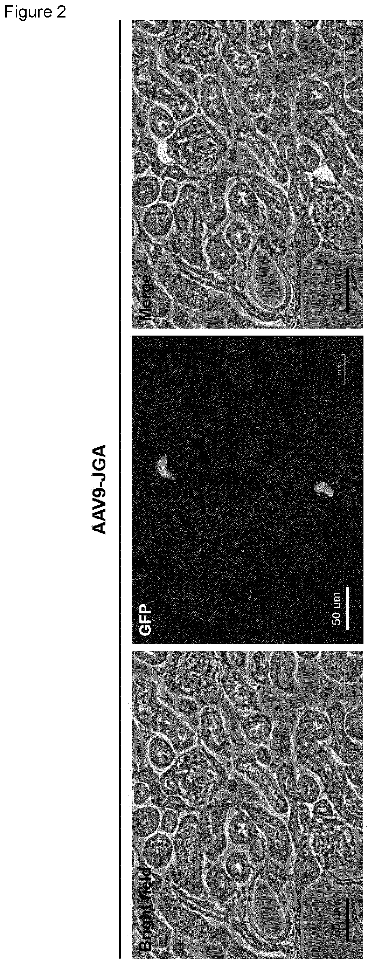

AAV9-JGA loaded with cDNA of green fluorescent protein (GFP) was intravenously administrated in mice to visualize transduced cells. Subsequent fluorescence microscopy showed a subset of the TAL adjacent to the JGA were transduced in the kidney by AAV9-JGA **(****Figure 2****).** The specificity was confirmed by co-staining with SLC12A1, which is a marker for the TAL **(****Figure 3****).** In addition, the GFP-positive cells are close to the MD marked by NOS1 and the GC marked by Renin, which are components of the JGA **(****Figure 3****).** According to the mosaic pattern of GFP-positive cells, it was further confirmed that they are a subset of TAL (CLDN16+/CLDN10-) cells **(****Figure 4****).** The AAV9-JGA transduced subset of the TAL is illustrated in a schema showing the structure and different cell types in the JGA in **Figure 5****.**

Further experiments demonstrated that this subset of TAL at JGA is susceptible to AAV transduction. AAV serotypes 1-9 and AAVrh10 loaded with cDNA of GFP were intravenously administrated in mice, respectively. Subsequent fluorescence microscopy showed all of the AAV serotypes transduced a subset of SLC12A1-positive TAL at JGA and the AAV transduced cells were negative for CLDN10 **(****Figure 6****).**

GFP delivered by AAV9-JGA exhibited no obvious signal in other organs, such as liver, heart and pancreas, which are regarded as off-targets, but GFP delivered by AAV9-WT showed strong signals in these off-target organs **(****Figure 7****).**

GFP delivered by AAV9-JGA exhibited signal for a long-term in the murine kidney. GFP was detected at 14, 60, 120, 180, 240 and 360 days after intravenous administration and maintained a consistent intensity **(****Figure 8****).**

The target transduction efficiency of AAV9-JGA was evaluated in rats. AAV9-JGA loaded with GFP cDNA was intravenously administrated in rats to visualize transduced cells. Subsequent fluorescence microscopy of the kidney showed the same subset of the TAL adjacent to glomerulus were transduced by AAV9-JGA (Figure 9), suggesting high transduction efficiency of AAV9-JGA in rat.

The peptide GGNYGLG was subcloned into the AAV serotype 2 or 8. AAV2-JGA or AAV8-JGA loaded with cDNA of green fluorescent protein (GFP) was intravenously administrated in mice to visualize transduced cells. Subsequent fluorescence microscopy showed an equal targeting specificity compared to AAV9-JGA (Figure 10), suggesting that targeting specificity mediated by the peptide GGNYGLG may be widespread in combination with other AAV serotypes.

Peptide GGNYGWG, GGNYGVG, GGNYGLV, GGNYDFG or GGNYALG, which were enriched in the on-target tissue and sharing the same motif with GGNYGLG, was cloned in AAV serotype 9. Recombinant vector loaded with cDNA of green fluorescent protein (GFP) was intravenously administrated in mice to visualize transduced cells. Subsequent fluorescence microscopy showed an equal targeting specificity compared to AAV9-JGA **(****Figure 11****),** indicating that the motifs with the sequences GGNYXX, GGNYXXX, GGNYGXX and GGNYXXG are of importance for the targeting specificity of the recombinant vector.

Taken together, these results suggest that AAV9-JGA can specifically and efficiently transduce the subset of the TAL adjacent to JGA in the kidney in mice and rats. Thus, this viral vector can be administered in a therapeutically effective amount to improve the dysfunction or disease related or caused by renal glomeruli, and to regulate the blood pressure.

### Methods

### Screening of AAV vector for targeting the kidney

An AAV vector comprising the targeting sequence as described herein may be obtained by screening using an in vivo selection protocol such as Korbelin et al. (2017)), adapted for the selection in the kidney. For example, an "AAV9 mutant (W503A) with peptide insertion site at amino acid position A589 (VP1 numbering) can be screened using the above-mentioned *in vivo* selection protocol (Korbelin et al. (2017)), being adapted for the selection in the kidney (Figure 1). In brief, C57BL/6J mice were intravenously injected with 1E+11 vg AAV9-W503A-A589 peptide library particles. Three days post-injection, mice were sacrificed and kidneys were harvested. Four screening rounds of the library were performed in vivo. In the first two rounds of screening, the AAV library fragments were rescued from the genomic DNA of the whole kidney. To increase the selection pressure, AAV library fragments were rescued from genomic DNA extracted from glomerulus and tubules in the third and fourth rounds of screening, respectively. To identify the most specific enriched AAV capsid candidates, three rating scores were established. The enrichment score 'E' was used to evaluate transduction efficacy in the target of each candidate reflecting changes in relative abundance from before-last to last selection. The general tissue specificity score 'GS' was used to assess the tropism of each candidate among target organs and other off-target organs (liver, heart, lung, muscle, brain, pancreas, and spleen) by multiplying the individual specificity scores. To determine the most promising candidate regarding specificity and efficacy, a combined score "C", was determined by multiplying E and GS.

A resulting variant comprises the target peptide sequence GGNYGLG (SEQ NO.: 2) as a partial sequence of each virion protein (VP), resulting in a total of 60 peptide copies in the fully assembled viral capsids. This variant is termed AAV9-JGA herein.

### Comparison of peptide motifs

By comparing AAV9-JGA with the peptide sequences, which were enriched in the on-target tissue, the following different sequences sharing the same motif were identified.
**GGNYG**W**G**
**GNYG**V**G**
**GGNYG**F**G**
**GGNYG**S**G**
**GGNYG**C**G**
**GGNYGGG**
**GGNYG**A**G**
**GGNYG**Y**G**
**GGNYG**WV
**GGNYG**LV
**GGNYG**LC
**GGNYG**LF
**GGNYG**MC
**GGNYG**LI
**GGNYG**GL
**GGNYG**VD
**GGNYG**FV
**GGNY**DFG
**GGNY**ALG
**GGNY**VLG
**GGNY**CLA

In summary, it was found that the several sequences (namely the sequences set out in SEQ ID NOs: 3 to 23) shared the motif GGNY to SEQ ID NO: 2, AAV9-JGA. Sampling inspection suggests that these peptide sequences have equal targeting specificity. Furthermore, it was found that the sequences with the SEQ ID NOs: 3 to 23 shared G at position 5 or G at position 7, which indicates their importance for the targeting specificity. Thus, the motifs with the sequences GGNYXX, GNYXXX, GGNYGXX and GGNYXXG (see SEQ ID NOs.: 1, 26, 24, and 25 respectively), where X is any natural amino acid, are of importance for the targeting specificity of the recombinant vector.

### Production of recombinant AAV vector

Recombinant AAV vectors were produced by plasmid co-transfection in HEK293T/17 cells using polyethylenimine (Polysciences, linear, MW 2500). All the recombinant AAV serotypes were generated with transgene either ssCMV-GFP-WPRE or scCMV-GFP (Addgene, plasmid 32396). The packaging plasmid AAV Rep2-Cap was co-transfected with an AAV2 ITRflanked transgene and adenoviral helper plasmids pXX6. Three days after transfections, cells and medium were harvested for Benzonase^{®} Nuclease (Sigma-Aldrich, E1014-25KU) treatment. AAV vectors were further purified by iodixanol gradient ultracentrifugation followed by dialysis and concentration against PBS using Amicon Ultra-15 centrifugal filter devices (Millipore, UFC910024). Physical particles were quantified by real-time PCR with the forward primer: 5'-GGGACTTTCCTACTTGGCA-3' and the reverse primer: 5'-GGCGGAGTTGTTACGACAT-3' directed to the CMV promoter sequence.

### Transduction of kidney cells

Intravenous injections of AAV vector were performed in 8-12 weeks old C57BL/6J mice and 6-8 weeks old Sprague Dewlay rats. AAV vectors expressing GFP reporter for tissue transduction efficiency and specificity evaluation were injected at the dose of 1E+11vg per mouse and 1E+12vg per rat. Two weeks post-injection, mice and rats were sacrificed and organs were harvested for histological analysis. The doses given to the mice of recombinant AAV serotype vectors expressing GFP are shown in the table below.

| **AAV serotypes** | **Dose vg/mouse** |
|---|---|
| scAAV 1 | 5.00E+11 |
| scAAV2 | 1.00E+11 |
| scAAV3 | 8.00E+10 |
| scAAV4 | 3.00E+11 |
| scAAV5 | 5.00E+11 |
| ssAAV6 | 3.00E+12 |
| ssAAV7 | 5.00E+11 |
| scAAV8 | 1.00E+11 |
| scAAV9 | 1.00E+11 |
| scAAVrh.10 | 1.00E+11 |

### Evaluation of cell specify

Kidney was perfused with 5 ml DPBS followed by 5 ml 4% PFA and subsequently fixed in 4% PFA at 4 °C overnight. For immunohistochemistry staining, kidney samples were washed with PBS three times and embedded in paraffin. Paraffin sections were stained with anti-GFP antibody 1:200 (Abcam, ab290) according to the ZytoChem Plus AP Polymer Kit (Zytomed Systems, POLAP-006) manufacturers' protocols. For the immunofluorescence staining, kidney samples were incubated in 15% sucrose in PBS at 4°C for 6 hours, followed by 30% sucrose in PBS overnight, and embedded in OCT. Cryosections were permeabilized with 0.1% Triton X-100. Nonspecific binding sites were blocked in 5% BSA supplemented with 0.3M Glycine in PBST. Cryosections were incubated overnight at 4°C with the following primary antibodies: chicken anti-GFP, 1:500 (Thermo Scientific, A10262); rabbit anti-SLC12A1, 1:200 (Abcam, ab171747); mouse anti-NOS1, 1:200 (Santa Cruz Biotechnology, sc-5302); rabbit anti-CLDN10, 1:200 (Thermo Scientific, 38-8400); rabbit anti-CLDN16, 1:100 (Bicell Scientific, 00216); rabbit anti-renin 1:200 (Abcam, ab212197). Alexa Fluor 488-, 594-, or 647-labeled secondary antibodies (Invitrogen or Thermo Fisher Scientific) was incubated at room temperature for 1 hour. DAPI was used for staining nuclei. For CLDN16 antibody staining, additional fixation was performed in methanol/acetone at -20 °C for 20 min. Tissues were antigen retrieved with boiled Target Retrieval Solution, Citrate pH6 for 30 min. Permeabilization, blocking, and antibody incubation were performed as described above.

The invention comprises the following items:
Item 1. Recombinant vector for targeting to kidney cells, comprising a targeting peptide comprising the sequence GGNYXX, wherein each X at position 5 to 6 is independently selected from any naturally occurring amino acid.
Item 2. Recombinant vector according to item 1, wherein the targeting peptide comprises at most 20 amino acids, preferably at most 10 amino acids, more preferably at most 9 amino acids, and even more preferably 8 amino acids.
Item 3. Recombinant vector according to item 2, wherein the targeting peptide comprises at most 7 amino acids.
Item 4. Recombinant vector according to any one of the preceding items, wherein X at position 5 is G.
Item 5. Recombinant vector according to any one of the preceding items, wherein X at position 7 is G.
Item 6. Recombinant vector according to any one of the preceding items, wherein the targeting peptide comprises the sequence GGNYGLG.
Item 7. Recombinant vector according to any one of the preceding items, wherein the targeting peptide is exposed to the surface of the recombinant vector.
Item 8. Recombinant vector according to any one of the preceding items, wherein the vector is an adeno associated virus (AAV) vector.
Item 9. Recombinant vector according to item 8, wherein the AAV is serotype AAV9.
Item 10. Recombinant vector according to any one of the preceding items, wherein the targeting peptide is incorporated into at least one capsid protein of the recombinant vector.
Item 11. Recombinant vector according to any one of the preceding items, wherein the targeting peptide is incorporated into each capsid protein of the recombinant vector.
Item 12. Recombinant vector according to any one of the preceding items, wherein the vector contains 60 copies of the targeting peptide.
Item 13. Recombinant vector according to any one of items 8 to 12, wherein the targeting peptide is introduced into the capsid protein after an amino acid residue corresponding to A589 of (virion protein 1) VP1 of AAV9.
Item 14. Recombinant vector according to any one of the preceding items, wherein the recombinant vector comprises a transgene packaged within the capsid.
Item 15. Nucleic acid encoding the recombinant vector according to any one of the preceding items.
Item 16. Recombinant vector according to any one of the preceding items, for use as a medicament.
Item 17. Recombinant vector according to any one of items 1-14, for use in the treatment of kidney diseases, preferably disorders related to juxtaglomerular apparatus, abnormal blood pressure and abnormal glomerular filtration rate.
Item 18. Recombinant vector for use according to items 16 to 17, wherein cells of the distal tubular epithelium adjacent to the kidney juxtaglomerular apparatus are specifically transduced.
Item 19. Recombinant vector for use according to items 16 to 18, wherein cells are positive for both markers SLC12A1 and CLDN16 are transduced.
Item 20. Recombinant vector for use according to items 16 to 19, wherein the cells are CLDN10 negative.
Item 21. Recombinant AAV capsid protein comprising the targeting peptide as defined in items 1 to 7.
Item 22. Nucleic acid encoding the recombinant AAV capsid protein defined in item 21.

### ABBREVIATIONS

- GFP: green fluorescent protein
- anti-GFP: antibody directed against GFP
- TAL: thick ascending limb of Henle's loop
- JGA: juxtaglomerular apparatus
- GFR: glomerular filtration rate
- DsRed: red fluorescent protein isolated from *Discosoma*
- AAV: adeno-associated virus
- AAV9: AAV vector of serotype 9
- FDA: US Food and Drug Administration
- MD: macula densa
- MC: mesangial cells
- SLC12A1: solute Carrier Family 12 Member 1
- NOS1: nitric oxide synthase 1
- DAPI: 4',6-diamidino-2-phenylindole
- RAAS: renin-angiotensin-aldosterone system
- RECA-1: anti-endothelial cell antibody
- WT: wild type
- VP: virion protein
- X: amino acid selected from any naturally occurring amino acid

### REFERENCES

Tenenbaum et al. (2003) Evaluation of risks related to the use of adeno-associated virus-based vectors. Curr Gene Ther 3, 545-565.
Rubin et al. (2020) Improving Molecular Therapy in the Kidney. Mol Diagn Ther 24, 375-396.
Bell et al. (2003) Macula densa cell signaling. Annu Rev Physiol 65, 481-500.
Korbelin et al. (2017) How to Successfully Screen Random Adeno-Associated Virus Display Peptide Libraries In Vivo. Hum Gene Ther Methods 28, 109-123.

## Claims

1. Recombinant vector for targeting to kidney cells, comprising a targeting peptide comprising the sequence GGNYXX, wherein each X at position 5 to 6 is independently selected from any naturally occurring amino acid.

2. Recombinant vector according to claim 1, wherein the targeting peptide comprises at most 20 amino acids, preferably at most 10 amino acids, more preferably at most 9 amino acids, and even more preferably 8 amino acids.

3. Recombinant vector according to claim 2, wherein the targeting peptide comprises at most 7 amino acids.

4. Recombinant vector according to any one of the preceding claims, wherein X at position 5 is G, and/or wherein X at position 7 is G.

5. Recombinant vector according to any one of the preceding claims, wherein the targeting peptide comprises the sequence GGNYGLG.

6. Recombinant vector according to any one of the preceding claims, wherein the targeting peptide is exposed to the surface of the recombinant vector.

7. Recombinant vector according to any one of the preceding claims, wherein the vector is an adeno associated virus (AAV) vector, optionally wherein the AAV is serotype AAV9.

8. Recombinant vector according to any one of the preceding claims, wherein the recombinant vector comprises a transgene packaged within the capsid.

9. Nucleic acid encoding the recombinant vector according to any one of the preceding claims.

10. Recombinant vector according to any one of the preceding claims, for use as a medicament.

11. Recombinant vector according to any one of claims 1 to 10, for use in the treatment of kidney diseases, preferably disorders related to juxtaglomerular apparatus, abnormal blood pressure and abnormal glomerular filtration rate.

12. Recombinant vector for use according to claims 10 to 11, wherein cells of the distal tubular epithelium adjacent to the kidney juxtaglomerular apparatus are specifically transduced.

13. Recombinant vector for use according to claims 11 to 12, wherein cells are positive for both markers SLC12A1 and CLDN16 are transduced, optionally wherein the cells are CLDN10 negative.

14. Recombinant AAV capsid protein comprising the targeting peptide as defined in claims 1 to 6.

15. Nucleic acid encoding the recombinant AAV capsid protein defined in claim 14.
